# EUROPEAN PATENT APPLICATION

(11) **EP 4 039 797 A1**
(43) Date of publication of application: **10.08.2022**
(21) Application number: 19813218.5
(22) Date of filing: 29.10.2019
(51) Int. Cl.: C12N 5/071

(54) **HUMAN FATTY LIVER MODEL CELLS**

(30) Priority: 26.08.2019 JP 2019153323
(71) Applicant: PhoenixBio Co., Ltd., Hiroshima 739-0046 (JP)
(72) Inventor: KAKUNI Masakazu, Higashi-Hiroshima-shi, Hiroshima 739-0046 (JP); TAKAHASHI Masaki, Higashi-Hiroshima-shi, Hiroshima 739-0046 (JP); HATA Keishi, Akita City, Akita 010-1623 (JP); TOMATSU Sayaka, Akita City, Akita 010-1623 (JP); SASAKI Akira, Akita City, Akita 010-1623 (JP); UMEKAWA Yui, Akita City, Akita 010-1623 (JP)
(74) Representative: Hoffmann Eitle
(86) International application number: PCT/JP2019/042317
(87) International publication number: WO 2021/038892

(57) **Abstract**

An object of the present invention is to provide human fatty-liver model cells showing symptoms of the hepatic tissue of fatty liver. The present invention relates to human fatty-liver model cells, which are produced by culturing human hepatocytes derived from fatty liver in a medium containing dimethyl sulfoxide.

## Description

### Technical Field

The present invention relates to a human fatty-liver model cells and a method for producing the model cells.

### Background Art

Fatty liver is a collective term referring to diseases producing liver disorder, which is caused by excessive accumulation of lipid such as neutral fat within hepatocytes. In fatty liver, accumulation of fat droplets is observed in a 1/3 or more area of the hepatocytes constituting the liver lobule. The occurrence of frequency of fatty liver likely increases year by year due to change in eating and lifestyle habits. In past days, it was considered that fatty liver may leave alone; however, in recent years, nonalcoholic fatty liver disease (NAFLD) has attracted attention; and cases where fatty liver develops into non-alcoholic steatohepatitis (NASH) and further into cirrhosis or liver cancer, have been found. Because of this, it has been required to suitably treat fatty liver. Attempts to functionally analyze its pathology and develop effective therapeutic agents have been made.

For investigating the onset mechanism of fatty liver and prevention and treatment thereof, non-human animal models exhibiting fatty-liver symptoms have been prepared. For example, Patent Literature 1 discloses that a non-human animal model exhibiting fatty-liver symptoms is prepared by transplanting human hepatocytes to an immunodeficient non-human animal with a liver disorder. In the non-human animal model, symptoms of fatty liver, such as large fat droplets and hepatic steatosis, are observed.

### Citation List

### Patent Literature

Patent Literature 1: WO2008/001614

### Summary of Invention

### Technical Problem

Non-human animal models have the following problems. A great deal of time and labor/cost are required for preparing, raising and managing the animal models. In addition, individual difference and reproducibility, and ethical limitations in use are also problems. For the reason, in order to efficiently investigate the onset mechanism of fatty liver and prevention and treatment for fatty liver, development of an *in vitro* evaluation system for human fatty liver, more specifically, human fatty-liver model cells, is strongly desired.

When the present inventors cultured human hepatocytes derived from fatty liver *in vitro,* they found that fat droplets disappear, and thus, the human hepatocytes cannot maintain the symptoms of fatty liver. More specifically, they excised out fatty liver from a non-human animal model showing the aforementioned symptoms of fatty liver, separated/collected human hepatocytes showing symptoms of fatty liver such as accumulation of fat droplets from the fatty liver, and cultured the human hepatocytes *in vitro.* As a result, they found that fat droplets disappear from the human hepatocytes, and thus, the human hepatocytes cannot maintain the symptoms of fatty liver.

Accordingly, an object of the present invention is to provide a novel method that enables human hepatocytes derived from fatty liver to maintain the symptoms of fatty liver such as accumulation of fat droplets, and provide novel human fatty-liver model cells.

### Solution to Problem

The present inventors conducted intensive studies with a view to attaining the aforementioned objects. As a result, accumulation of fat droplets, lipid secretion and/or accumulation, expression of fatty liver related genes and others were observed by culturing human hepatocytes derived from fatty liver in a medium containing dimethyl sulfoxide. They found that human fatty-liver model cells maintaining the symptoms of fatty liver can be obtained.

The present invention was attained based on these findings and has the following features.
[1] A method for producing human fatty-liver model cells, including a step of culturing human hepatocytes derived from fatty liver in a medium containing dimethyl sulfoxide.
[2] The method according to [1], in which the human hepatocytes derived from fatty liver are collected from a chimeric non-human animal having human hepatocytes.
[3] The method according to [1] or [2], in which culture is carried out for more than 3 days.
[4] Human fatty-liver model cells that secrete and/or accumulate lipid.
[5] The cells according to [4], containing a lipoprotein including a very low density lipoprotein (VLDL) and a low density lipoprotein (LDL), in which VLDL is contained more than LDL.
[6] The cells according to [4], having increased expression of a fatty liver related gene.
[7] The cells according to [6], in which the fatty liver related gene is at least one gene selected from the group consisting of FASN, SREBF1 and G6PC.
[8] A method for screening for a substance effective for human fatty liver, including the steps of:
   administering a test substance to the cells according to any one of claims 4 to 7; and
   comparing severity of fatty-liver symptoms between cells to which the test substance is administered and cells to which the test substance is not administered.
[9] A method for evaluating toxicity of a test substance to human fatty liver, including the steps of:
   administering a test substance to the cells according to any one of claims 4 to 7; and
   comparing survival rate and severity of fatty-liver symptoms between the cells to which the test substance is administered and cells to which the test substance is not administered, to evaluate effect of the test substance on human fatty liver.

The matters disclosed in the description and/or the drawings of Japanese Patent Application No. 2019-153323 as a basis of priority to the present application are incorporated herein.

All the publications, patents and patent applications cited herein are incorporated herein by reference in their entirety.

### Advantageous Effects of Invention

According to the present invention, it is possible to provide human fatty-liver model cells in which, e.g., accumulation of fat droplets, secretion and/or accumulation of lipid and expression of fatty liver related genes are observed, and a method for producing the cells.

### Brief Description of Drawings

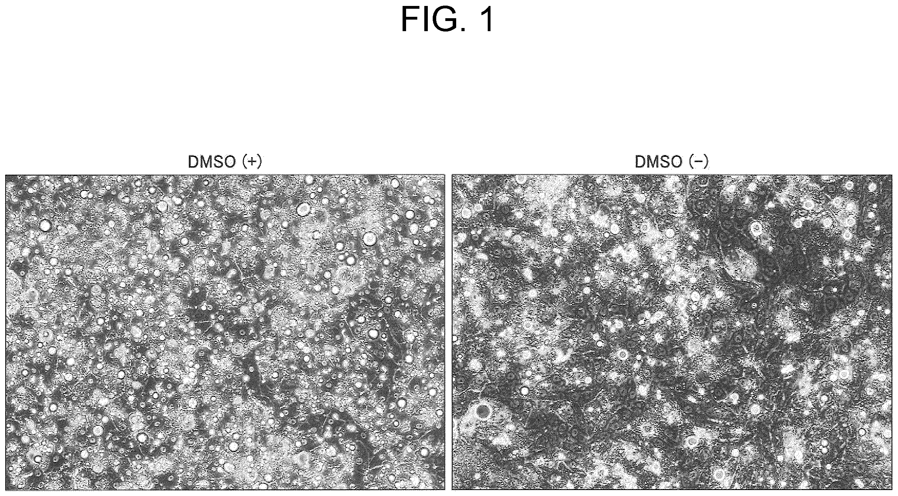

[Figure 1] Figure 1 shows photographs of PXB-cells cultured in medium B (DMSO (+)) or medium C (DMSO (-)) for 5 days. The left photograph shows PXB-cells cultured in medium B (DMSO (+)) and the right photograph shows PXB-cells cultured in medium C (DMSO (-)).

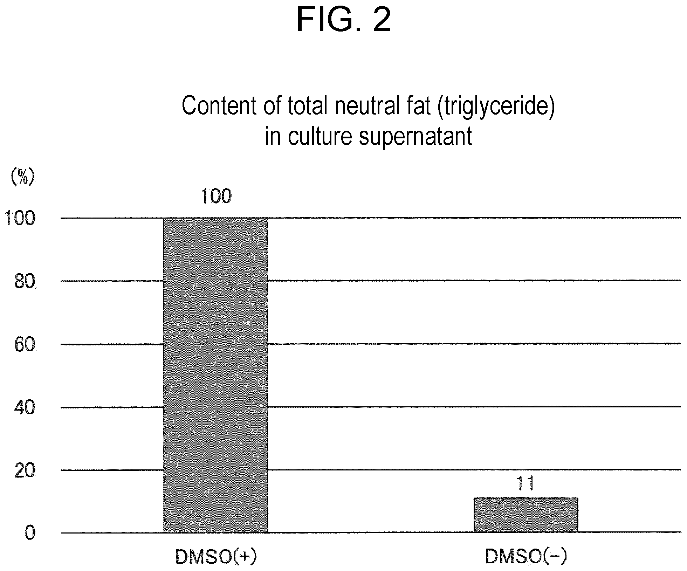

[Figure 2] Figure 2 shows the measurement results of the content of total neutral fat (triglyceride) of lipoproteins contained in each of the culture supernatants of PXB-cells cultured in medium B (DMSO (+)) and medium C (DMSO (-)) for 5 days. The results are shown by relative values based on the content (regarded as "100") of the total neutral fat in the culture supernatant of PXB-cells cultured in medium B (DMSO (+)).

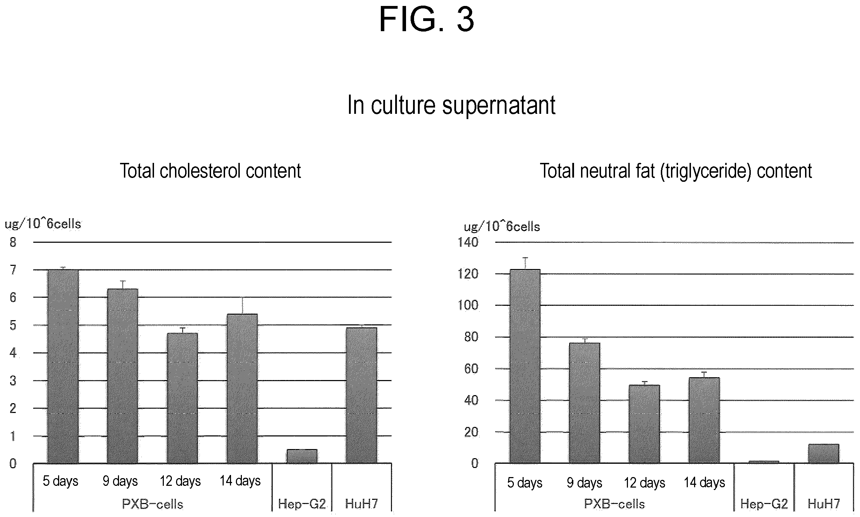

[Figure 3] Figure 3 shows the measurement results of the contents of total cholesterol (left) and total neutral fat (triglyceride) (right) of lipoproteins in each of culture supernatants of PXB-cells cultured in medium B (DMSO (+)) for 5 days, 9 days, 12 days and 14 days, and HepG2 cells and HuH7 cells.

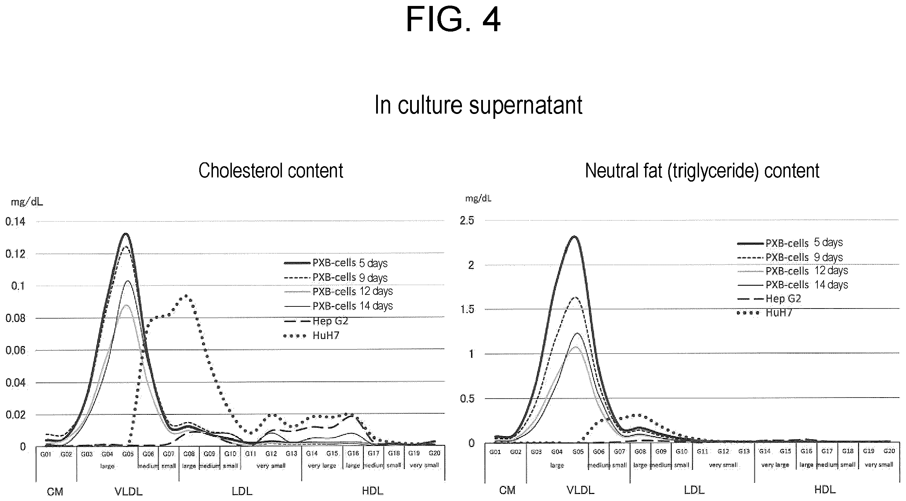

[Figure 4] Figure 4 shows the analysis results of the contents of cholesterol (left) and neutral fat (triglyceride) (right) of lipoproteins (4 types of subgroups) in each of culture supernatants of PXB-cells cultured in medium B (DMSO (+)) for 5 days, 9 days, 12 days and 14 days, and HepG2 cells and HuH7 cells.

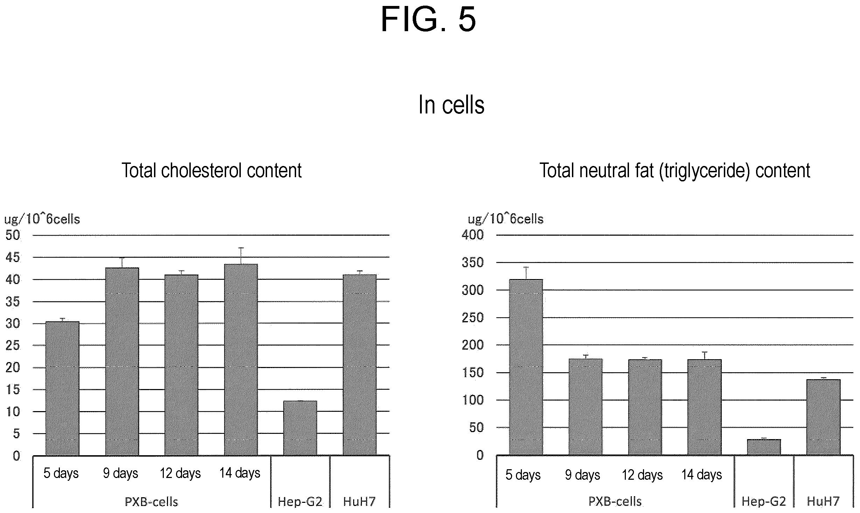

[Figure 5] Figure 5 shows the measurement results of the contents of total cholesterol (left) and total neutral fat (triglyceride) (right) within PXB-cells cultured in medium B (DMSO (+)) for 5 days, 9 days, 12 days and 14 days, and HepG2 cells and HuH7 cells.

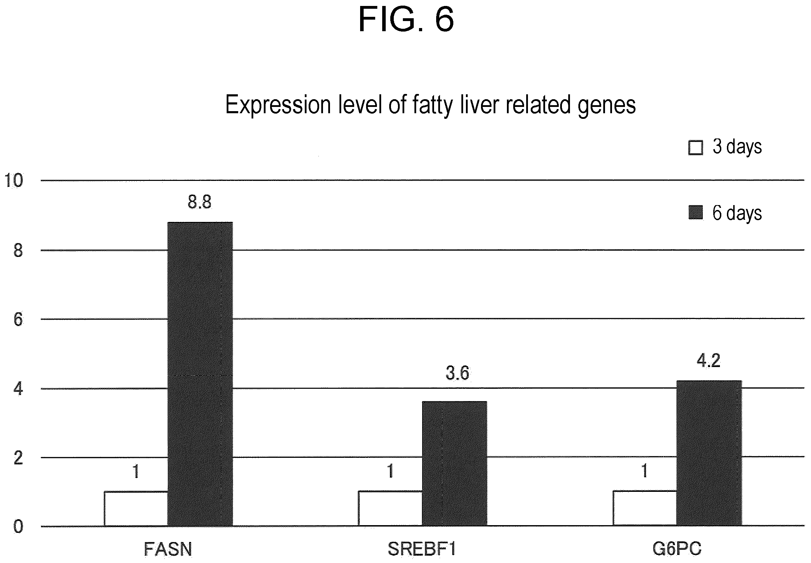

[Figure 6] Figure 6 shows the measurement results of expression levels of fatty liver related genes (FASN, SREBF1, G6PC) in PXB-cells cultured in medium B (DMSO (+)) for 3 days and 6 days. The results are shown by relative values based on the expression level (regarded as "1") of each of the genes of PXB-cells cultured in medium B (DMSO (+)) for 3 days.

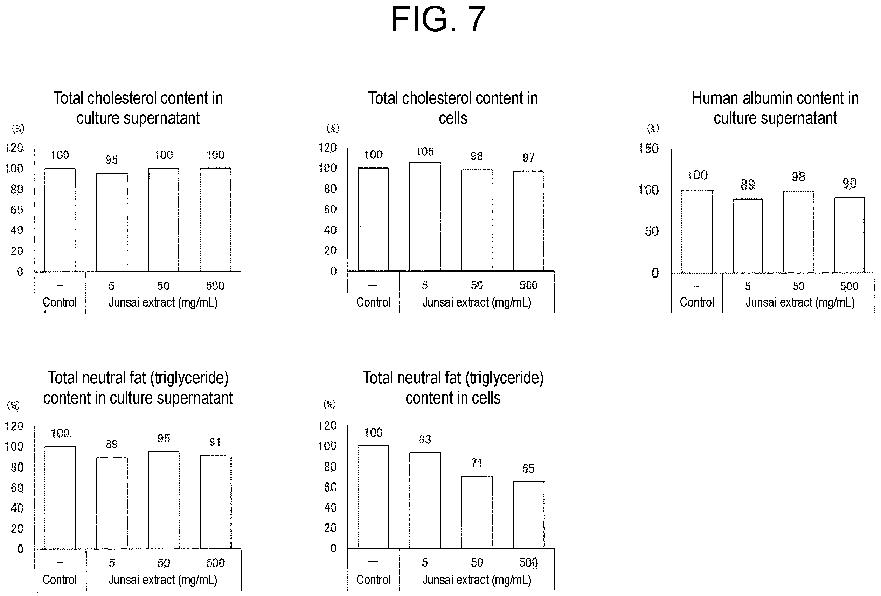

[Figure 7] Figure 7 shows the measurement results of the total cholesterol content and total neutral fat (triglyceride) content in cells and in the culture supernatants of PXB-cells cultured in medium B (DMSO (+)) supplemented with a junsai (water shield) extract (5 µg/mL, 50 µg/mL, 500 µg/mL) for 2 days and the content of human albumin in the culture supernatants. The results are shown by relative values based on the contents (regarded as "100") of the total cholesterol, total neutral fat (triglyceride) and human albumin in the control.

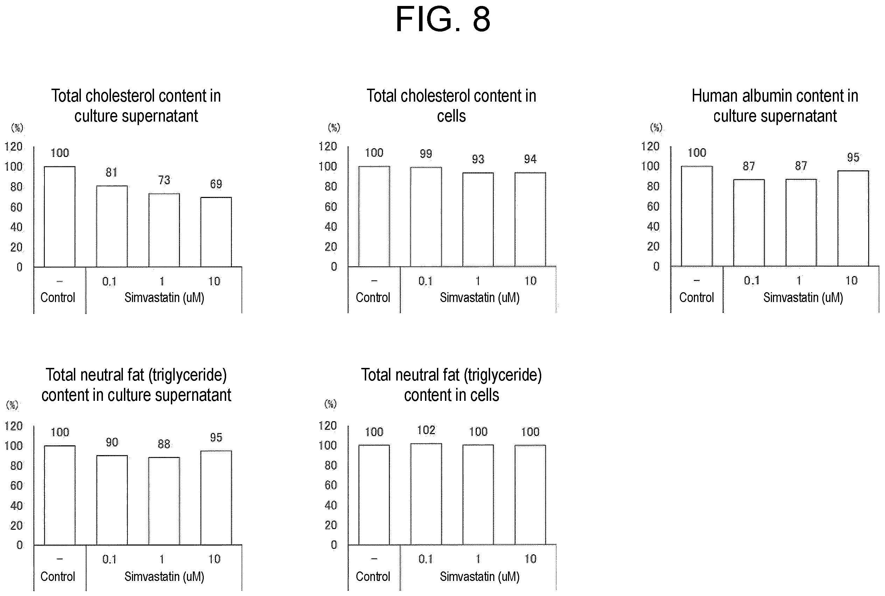

[Figure 8] Figure 8 shows the measurement results of the total cholesterol content and total neutral fat (triglyceride) content in cells and in the culture supernatants of PXB-cells cultured in medium B (DMSO (+)) supplemented with simvastatin (0.1 µM, 1 µM or 10 µM) for 2 days and the content of human albumin in the culture supernatants. The results are shown by relative values based on the contents (regarded as "100") of the total cholesterol, total neutral fat (triglyceride) and human albumin in the control.

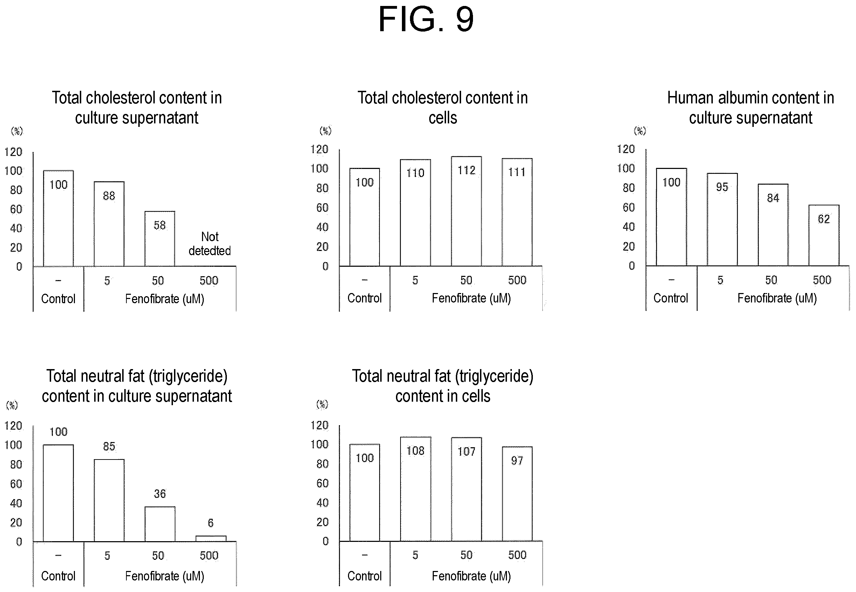

[Figure 9] Figure 9 shows the measurement results of the total cholesterol content and total neutral fat (triglyceride) content in cells and in the culture supernatants of PXB-cells cultured in medium B (DMSO (+)) supplemented with fenofibrate (5 µM, 50 µM or 500 µM) for 2 days and the content of human albumin in the culture supernatants. The results are shown by relative values based on the contents (regarded as "100") of the total cholesterol, total neutral fat (triglyceride) and human albumin in the control.

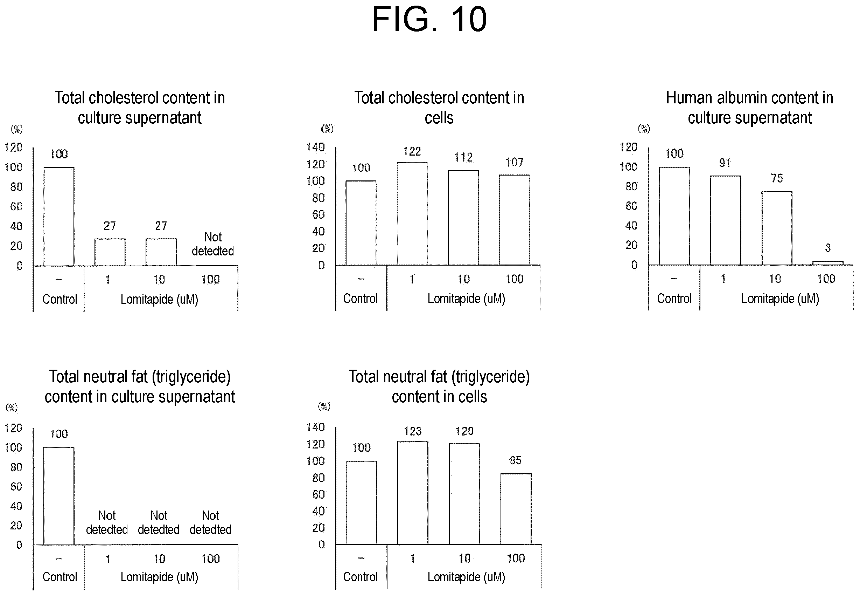

[Figure 10] Figure 10 shows the measurement results of the total cholesterol content and total neutral fat (triglyceride) content in cells and in the culture supernatants of PXB-cells cultured in medium B (DMSO (+)) supplemented with lomitapide (1 µM, 10 µM or 100 µM) for 2 days and the content of human albumin in the culture supernatants. The results are shown by relative values based on the contents (regarded as "100") of the total cholesterol, total neutral fat (triglyceride) and human albumin in the control.

### Description of Embodiments

### 1. Method for producing human fatty-liver model cells

The present invention relates to a method for producing human fatty-liver model cells, including a step of culturing human hepatocytes derived from fatty liver in a medium containing dimethyl sulfoxide.

### [1-1] Human hepatocytes derived from fatty liver

In the present invention, "human hepatocytes derived from fatty liver" refer to human hepatocytes collected from a fatty liver tissue. The human hepatocytes can be once frozen and then thawed to put in use. The fatty liver tissue that can be used includes a fatty liver tissue derived from a human patient with fatty liver and a fatty liver tissue derived from a non-human animal model (hereinafter referred to as a "chimeric non-human animal"), which is obtained by transplanting human hepatocytes to an immunodeficient non-human animal with liver disorder.

Human hepatocytes can be collected from a fatty liver tissue in accordance with a method known in the art such as a collagenase perfusion method by use of a means such as a centrifuge, an elutriator, FACS (fluorescence activated cell sorter) and a monoclonal antibody specifically recognizing human hepatocytes. The "human hepatocytes derived from fatty liver" of the present invention are preferably human hepatocytes collected from a chimeric non-human animal, in view of large-scale production and stable supply.

### [1-2] Human hepatocytes collected from chimeric non-human animal

The human hepatocytes collected from a fatty liver tissue derived from a chimeric non-human animal available in the present invention can be prepared in accordance with the following method.

### • 1-2-1 Chimeric non-human animal

In the present invention, the "chimeric non-human animal" refers to a non-human animal having hepatocytes of the liver partly or wholly replaced with human hepatocytes.

The "non-human animal" is preferably a mammal and more preferably a rodent. Examples of the rodent include a mouse, a rat, a guinea pig, a squirrel and a hamster. Of them, a mouse or rat generally used as an experimental animal is particularly preferable.

A chimeric non-human animal having human hepatocytes can be obtained by transplanting human hepatocytes to an immunodeficient non-human animal with a liver disorder in accordance with a method known in the art (Japanese Patent Laid-Open No. 2002-45087, WO2008/001614, WO2013/145331).

### • 1-2-2 Immunodeficient non-human animal with a liver disorder

The "immunodeficient non-human animal with a liver disorder" refers to an animal being immunodeficient (showing no rejection response to xenogeneic cells) and having a damage in hepatocytes derived from a non-human animal. Since hepatocytes derived from a non-human animal are damaged, human hepatocytes transplanted easily proliferate and also the function of the liver can be maintained by the human hepatocytes transplanted.

An immunodeficient non-human animal with a liver disorder can be prepared by applying a liver-disorder induction treatment and an immunodeficiency induction treatment to a same individual. Examples of the "liver-disorder induction treatment" include administration of a liver-disorder induction substance (for example, carbon tetrachloride, yellow phosphorus, D-galactosamine, 2-acetylaminofluorene, pyrrolizidine alkaloid) and a surgical treatment (for example, partial excision of the liver). Examples of the "immunodeficiency induction treatment" include administration of an immunosuppressant and excision of the thymus.

Alternatively, the immunodeficient non-human animal with a liver disorder can be prepared by applying a liver-disorder induction treatment to a genetically immunodeficient animal. Examples of the genetically immunodeficient animal that can be used include a severe combined immunodeficient (SCID) animal showing T cell system failure, an animal losing T cell function due to genetic defect of the thymus and a RAG2 gene knockout animal. Specific examples thereof that can be used include SCID mouse, NUDE mouse, RAG2 knockout mouse, IL2Rgc/Rag2 knockout mouse, NOD mouse, NOG mouse, nude mouse, nude rat and an immunodeficient rat, which is obtained by transplanting the bone marrow of an SCID mouse to an X irradiated nude rat (Japanese Patent Laid-Open No. 2007-228962, Transplantation, 60 (7): 740-7, 1995).

Alternatively, the immunodeficient non-human animal with a liver disorder can be prepared by applying an immunodeficiency induction treatment to an animal genetically having a liver disorder. As the animal genetically having a liver disorder, a transgenic animal, which is obtained by introducing a liver-disorder inducing protein-encoding gene ligated under control of an enhancer and/or promoter for a hepatocyte-specifically expressed protein, can be used. Examples of the "hepatocyte-specifically expressed protein" include serum albumin, cholinesterase and Hageman factor. Enhancers and/or the promoters for controlling expression of these genes can be used. Examples of the "liver-disorder inducing protein" include an urokinase plasminogen activator (uPA) and a tissue plasminogen activator (tPA). In the transgenic animal as mentioned above, since a liver-disorder inducing protein is expressed specifically to hepatocytes under control of an enhancer and/or promoter for a hepatocyte-specifically expressed protein, a liver disorder is induced. Alternatively, the animal genetically having a liver disorder can be prepared by knockout of a gene responsible for liver function. Examples of the "gene responsible for liver function" include a fumarylacetoacetate hydrase gene.

Alternatively, the immunodeficient non-human animal with a liver disorder can be prepared by crossing a genetically immunodeficient animal with an animal of the same species genetically having a liver disorder.

Alternatively, the immunodeficient non-human animal with a liver disorder can be prepared by introducing a genetic factor causing immunodeficiency and/or a liver disorder as mentioned above to a non-human animal, a fertilized egg derived from a non-human animal having genetic immunodeficiency and/or genetic liver disorder, or pluripotent stem cells (for example, embryonic stem cells (ES cells) and induced pluripotent stem cells (iPS cells)), by using a genome editing technique and a genetic engineering technique, such as gene targeting CRISPR-Cas9, zinc finger nuclease (ZFN) and TALE nuclease (TALEN) (Wang, H. et al., Cell, 153, 910-918, (2013); Yang, H. et al., Cell, 154, 1370-1379, (2013)).

In the present invention, the "immunodeficient non-human animal with a liver disorder" may have a gene specifying a trait of immunodeficiency and a gene specifying a trait of a liver disorder, each in a homozygous state or heterozygous state. As the immunodeficient non-human animal with a liver disorder of the present invention, for example, liver disorder immunodeficient mice having a genotype represented by, e.g., uPA (+/-)/SCID (+/+) and uPA (+/+)/SCID (+/+), can be suitably used.

### • 1-2-3 Human hepatocytes to be transplanted

In the present invention, the "human hepatocytes" to be transplanted to an immunodeficient non-human animal with a liver disorder may be any hepatocytes as long as they are derived from a human; for example, human hepatocytes isolated from a human liver tissue by a routine method such as a collagenase perfusion method can be used. The human liver tissue may be a liver tissue derived from a healthy person or derived from a patient affected with a disease such as fatty liver and liver cancer; however, a liver tissue derived from a healthy person is preferable. The age of the person from which hepatocytes are to be isolated is not particularly limited; however, the hepatocytes are preferably isolated from a liver tissue of a child not more than 14 years old. If hepatocytes taken from a child not more than 14 years old are used, a high replacement rate with the human hepatocytes after transplantation can be attained. The hepatocytes isolated can be once frozen and thawed and then put in use.

The human hepatocytes may be proliferative human hepatocytes capable of actively proliferating *in vivo.* The "proliferative human hepatocytes" refer to human hepatocytes forming colonies of a single cell type as a group and proliferating in such a manner that the size of a colony is increased under *in-vitro* culture conditions. The proliferation is sometimes called as "clonal proliferation", for the reason that the cells constituting colonies belong to a same type. The number of such cells can be further increased by subculture.

As the proliferative human hepatocytes, human small hepatocytes are mentioned (Japanese Patent Laid-Open No. H08-112092; Japan Patent No. 3266766; U.S. Patent No. 6,004,810, Japanese Patent Laid-Open No. H10-179148: Japan Patent No. 3211941, Japanese Patent Laid-Open No. H7-274951; Japan Patent No. 3157984, Japanese Patent Laid-Open No. H9-313172; Japan Patent No. 3014322).

The human hepatocytes isolated may be directly used or further purified and then put in use. Hepatocytes can be purified in accordance with a routine method by use of a means such as a centrifuge, an elutriator, FACS and a monoclonal antibody specifically recognizing hepatocytes which proliferate while forming colonies. As the monoclonal antibody specifically recognizing human hepatocytes and proliferative human hepatocytes, those known in the art (WO2008/001614) can be used.

Examples of the human hepatocytes that can be also used include human hepatocytes isolated from a liver tissue of a chimeric non-human animal having human hepatocytes in accordance with a routine method such as a collagenase perfusion method, the human hepatocytes once frozen and thawed, human hepatocytes obtained by induction of pluripotent stem cells (for example, embryonic stem cells (ES cells) and induced pluripotent stem cells (iPS cells)), hepatic progenitor cells such as Clip cells, human hepatocytes proliferated *in vitro,* cryopreserved hepatocytes, hepatocytes immortalized by introduction of, e.g., a telomerase gene and a mixture of these hepatocytes and non-parenchymal cells.

### • 1-2-4 Transplantation of human hepatocytes

Human hepatocytes can be transplanted to the liver of an immunodeficient non-human animal with a liver disorder via the spleen of the non-human animal or (directly) through the portal vein. The number of human hepatocytes to be transplanted can be about 1 to 2,000,000 and preferably 200,000 to 1,000,000. The gender of the immunodeficient non-human animal with a liver disorder is not particularly limited. Also, the age in days of the immunodeficient non-human animal with a liver disorder to be used for transplantation is not particularly limited; however, an animal of about 0 to 40 days after birth and preferably about 8 to 40 days after birth can be used because human hepatocytes, which are transplanted to an animal of an early age, more actively proliferate with a growth of the animal.

The animal transplanted with human hepatocytes can be raised in accordance with a routine method. For example, if the animal is raised for about 40 to 200 days after transplantation, a chimeric non-human animal having hepatocytes of the non-human animal partly or wholly replaced with human hepatocytes, can be obtained. In the liver of the chimeric non-human animal thus obtained, the symptoms of fatty liver, such as large fat droplets and hepatic steatosis, are observed (WO2008/001614).

### • 1-2-5 Recovery of human hepatocytes

Human hepatocytes are collected from a chimeric non-human animal in accordance with a routine method such as a collagenase perfusion method. Human hepatocytes are preferably collected by using a chimeric non-human animal having a high content of human hepatocytes in the hepatocytes to be collected; for example, using a chimeric non-human animal having one or more of the following features.
(i) 60% or more, preferably 70% or more, more preferably 80% or more, further preferably 90% or more, further more preferably 95% or more and particularly preferably 99% or more of the hepatocytes in the liver are replaced by human hepatocytes;
(ii) The blood human albumin level is 0.1 mg/mL or more, preferably 0.5 mg/mL or more, more preferably 1 mg/mL or more, further preferably 5 mg/mL or more and further more preferably 10 mg/mL or more;
(iii) 12 to 21 weeks, preferably 13 to 20 weeks, more preferably 14 to 19 weeks have passed after transplantation of human hepatocytes.

The human hepatocytes collected may be directly used. Alternatively, the human hepatocytes may be purified by use of a monoclonal antibody specifically recognizing human hepatocytes or hepatocytes of a non-human animal and put in use. When the hepatocytes isolated are reacted with a human hepatocyte-specific monoclonal antibody, the cells bound to the antibody are recovered by a flow cytometer (FACS) or a magnetic cell separator (MACS). Alternatively, when the hepatocytes isolated are reacted with a monoclonal antibody specific to non-human animal hepatocytes, the cells not bound to the antibody are recovered by means of FACS or MACS. In this manner, human hepatocytes can be purified and collected.

The human hepatocytes collected are further transplanted to another immunodeficient non-human animal with a liver disorder (passage transplant) in the same manner as above, and thereafter, may be collected in the same manner as above. The passage transplant can be carried out once or a plurality of times (for example, 2 to 4 times).

### [1-3] Culture of human hepatocytes derived from fatty liver

In the present invention, human hepatocytes derived from fatty liver can be cultured using a medium generally used for culturing animal cells. Examples of the medium include, but are not limited to, Dulbecco's modified eagle medium (DMEM) and Williams medium E. DMEM can be preferably used. In the medium, if necessary, further, e.g., fetal bovine serum, insulin, an epidermal growth factor, dexamethasone, a buffer, an antibiotic substance, a pH regulator, proline, ascorbic acid and nicotinamide can be appropriately added.

In the medium, dimethyl sulfoxide (DMSO) is added. DMSO can be added such that a final concentration thereof becomes 1 to 4 wt% and preferably 1 to 2 wt%; for example, 2 wt%. Due to addition of DMSO in a medium, a function of the human hepatocytes derived from fatty liver to absorb and/or secrete lipid can be enhanced to maintain accumulation of the lipid.

The human hepatocytes derived from fatty liver are seeded in a medium in an amount of 0.21 to 21.3 × 10⁵ cells/cm² and preferably 1.07 to 3.2 × 10⁵ cells/cm²; for example, 2.13 × 10⁵ cells/cm². If the amount of cells is less than 0.21 cells/cm², a sufficient amount of cells serving as human fatty-liver model cells cannot be obtained, in some cases. In contrast, if the amount is more than 21.3 × 10⁵ cells/cm², for example, growth of the cells decreases, secretion and/or accumulation amount of lipid decreases, in some cases.

The culturing of human hepatocytes derived from fatty liver may be carried out for a sufficient period for the cells to secrete and/or accumulate lipid; for example, the culturing can be carried out for more than 3 days, preferably 4 days or more and further preferably 5 days or more. The upper limit of the culture period is not particularly limited; for example, the upper limit can be 17 days or less and preferably 13 days or less. The medium can be appropriately exchanged in the culture period.

After completion of culture, human hepatocytes secreting and/or accumulating lipid can be used as the human fatty-liver model cells.

### 2. Human fatty-liver model cells

### [2-1] Secretion amounts of fat droplets and lipoprotein

The present invention also relates to human fatty-liver model cells, which are cultured hepatocytes derived from a human and secreting and/or accumulating a large amount of lipid, similarly to the hepatocytes in the human fatty liver.

The human fatty-liver model cells of the present invention contain a large number of fat droplets therein and have a high content and/or secretion amount of lipoproteins. The phrase "contain a large number of fat droplets therein" herein refers to containing fat droplets 2 times or more, 3 times or more, 4 times or more or 5 times or more, preferably 6 times or more, more preferably 7 times or more, further preferably 8 times or more and further more preferably 9 times or more as large as in the human hepatocytes derived from fatty liver cultured for more than 3 days, preferably 4 days or more and further preferably 5 days or more in human hepatocytes derived from fatty liver cultured in the same medium except that DMSO is not contained. The amount of fat droplets within the cells can be quantified by staining the fat droplets within the cells in accordance with a method known in the art such as oil red O staining, followed by extracting the pigment with an organic solvent. The "lipoprotein" is a composite particle for transporting a lipid such as cholesterol and neutral fat from an absorption/synthesis site to an application site, and having a structure consisting of a hydrophilic substance such as a phosphorus lipid, free cholesterol and apolipoprotein arranged on the outer side and a hydrophobic substance such as cholesterol and neutral fat arranged on the inner side. The "high content and/or secretion amount of lipoproteins" refers to containing lipoproteins in an amount 5 times or more, preferably 6 times or more, more preferably 7 times or more, further preferably 8 times or more and further more preferably 9 times or more as large as lipoproteins (more preferably triglyceride) contained in human hepatocytes derived from fatty liver, which are cultured for more than 3 days, preferably 4 days or more and further preferably 5 days or more in human hepatocytes derived from fatty liver cultured in the same medium except that DMSO is not contained, or contained in the culture supernatant thereof. The amount of lipoproteins contained in cells or culture supernatant can be measured by a method known in the art as described later.

### [2-2] Content of lipoprotein subclass

The human fatty-liver model cells of the present invention can be characterized also by the content of a lipoprotein subclass in cells or culture supernatant.

Lipoproteins can be classified into several subclasses in accordance with difference in properties such as the size, hydration density and electrophoretic mobility of particles. In the present invention, lipoproteins can be roughly classified into 4 groups: chylomicron (CM), very low density lipoprotein (VLDL), low density lipoprotein (LDL) and high density lipoprotein (HDL), based on the particle sizes described below, in accordance with a method known in the art (WO2007/052789). According to the method, CM is further divided into 2 subclasses; VLDL into 5 subclasses; LDL into 6 subclasses; and HDL into 7 subclasses. In short, the lipoproteins herein can be classified, in total, into 20 subclasses.

**[Table 1]**

| Subclass | Particle size |
|---|---|
| Chylomicron (CM) | Beyond 64 nm |
| Very low density lipoprotein (VLDL) | 31.3 nm or more and 64 nm or less |
| Low density lipoprotein (LDL) | 16.7 nm or more and less than 31.3 nm |
| High density lipoprotein (HDL) | 7.6 nm or more and less than 16.7 nm |

Subclasses of lipoproteins in cells or in culture supernatant can be quantified by fractionation by a method known in the art (WO2007/052789; Japanese Patent Laid-Open No. H9-15225; Arterioscler Thromb Vasc Biol. 2005; 25: 1-8; LipoSEARCH (registered trademark) (Skylight Biotech, Inc.)) using gel filtration liquid chromatography.

In the cells or culture supernatant of the human fatty-liver model cells of the present invention, the content of VLDL among lipoproteins is the highest.

In the cells or culture supernatant of the human fatty-liver model cells of the present invention, the content of VLDL is higher than that of LDL, for example, 2 times or more, 3 times or more, 4 times or more, 5 times or more, 6 times or more, 7 times or more, 8 times or more, 9 times or more, 10 times or more or 15 times or more as high as that of LDL.

More specifically, in the cells or culture supernatant of the human fatty-liver model cells of the present invention, the content of cholesterol of VLDL is higher than that of cholesterol of LDL, for example, 2 times or more, 3 times or more, 4 times or more, 5 times or more, 6 times or more, 7 times or more, 8 times or more, 9 times or more, 10 times or more or 15 times or more as high as that of cholesterol of LDL; and the content of neutral fat (triglyceride) of VLDL is higher than that of neutral fat (triglyceride) of LDL, for example, 2 times or more, 3 times or more, 4 times or more, 5 times or more, 6 times or more, 7 times or more, 8 times or more, 9 times or more, 10 times or more or 15 times or more as high as that of neutral fat (triglyceride) of LDL.

Further, in the cells or culture supernatant of the human fatty-liver model cells of the present invention, the content of VLDL is higher than that of HDL, for example, 5 times or more, 10 times or more, 15 times or more, 20 times or more, 25 times or more, 30 times or more, 40 times or more, 50 times or more, 60 times or more, 70 times or more, 80 times or more or 90 times or more as high as that of HDL.

More specifically, in the cells or culture supernatant of the human fatty-liver model cells of the present invention, the content of cholesterol of VLDL is higher than that of cholesterol of HDL, for example, 5 times or more, 10 times or more, 15 times or more, 20 times or more, 25 times or more, 30 times or more, 40 times or more, 50 times or more, 60 times or more, 70 times or more, 80 times or more or 90 times or more as high as that of cholesterol of HDL; and the content of neutral fat (triglyceride) of VLDL is higher than that of neutral fat (triglyceride) of HDL, for example, 5 times or more, 10 times or more, 15 times or more, 20 times or more, 25 times or more, 30 times or more, 40 times or more, 50 times or more, 60 times or more, 70 times or more, 80 times or more or 90 times or more as high as that of neutral fat of HDL.

In the cells or culture supernatant of the human fatty-liver model cells of the present invention, the content of LDL is higher than that of HDL, for example, 2.5 times or more, 3 times or more, 4 times or more, 5 times or more, 6 times or more, 7 times or more, 8 times or more, 9 times or more or 10 times or more as high as that of HDL.

More specifically, in the cells or culture supernatant of the human fatty-liver model cells of the present invention, the content of cholesterol of LDL is higher than that of cholesterol of HDL, for example, 2.5 times or more, 3 times or more, 4 times or more, 5 times or more, 6 times or more, 7 times or more, 8 times or more, 9 times or more or 10 times or more as high as that of cholesterol of HDL; and the content of neutral fat (triglyceride) of LDL is higher than that of neutral fat (triglyceride) of HDL, for example, 2.5 times or more, 3 times or more, 4 times or more, 5 times or more, 6 times or more, 7 times or more, 8 times or more, 9 times or more or 10 times or more as high as that of neutral fat (triglyceride) of HDL.

### [2-3] Expression level of fatty liver related gene

The human fatty-liver model cells of the present invention can be characterized also by the expression levels of fatty liver related genes in the cells.

In the present invention, the "fatty liver related genes" refer to genes the expression of which increases in the hepatocytes of fatty liver compared in hepatocytes of healthy liver. Examples of the fatty liver related genes include a gene encoding fatty acid synthase (gene name: FASN), a gene encoding SREBP-1 (gene name: SREBF1), a gene encoding glucose-6-phosphatase (G6PC), a gene encoding cholesterol 7α-hydroxylase (CYP7A1), a gene encoding a cholesteryl ester transfer protein (CETP), a gene encoding glucokinase (GCK) and a gene encoding phosphoenolpyruvate carboxykinase 1 (PCK1). The "high" expression levels of fatty liver related genes means that the expression levels of fatty liver related genes are high compared to those in human hepatocytes derived from fatty liver cultured in a DMSO-free medium or in a DMSO-containing medium for 3 days or less, for example, 2 times or more, preferably 3 times or more, more preferably 3.5 times or more as high as those.

The expression level of the fatty liver related genes can be quantified by a method known in the art, and preferably microarray analysis.

### [2-4] Others

The human fatty-liver model cells of the present invention can be produced by the aforementioned method for producing human fatty-liver model cells.

The human fatty-liver model cells of the present invention are preferably cultured in medium containing DMSO.

The human fatty-liver model cells of the present invention, since the content of VLDL among the lipoproteins is the largest, can be used as a model analogous to the hepatocytes of human fatty liver, compared to the hepatocytes (e.g., HepG2, HuH7) known in the art.

The human fatty-liver model cells of the present invention can be used as a human fatty liver model. Although the use of the human fatty-liver model cells is not particularly limited, the model cells can be used in a screening method for a substance effective for human fatty liver. The screening can be made by administering a test substance to a culture of the human fatty-liver model cells of the present invention and comparing the severity of fatty-liver symptoms between cells to which the test substance is administered and cells to which the test substance is not administered. The "cells to which the test substance is administered and cells to which the test substance is not administered" may be the same culture before and after administration of the test substance or separate cultures obtained in the same procedure except the presence or absence of the test substance. Examples of the "fatty-liver symptoms" include, but not limited to, accumulation of fat droplets, secretion and/or accumulation of lipid, expression of fatty liver related genes, iron deposition, apoptosis, expression of a protein causing oxidative stress, balloon swelling (ballooning) and Mallory body. In the cells to which a test substance is administered, if these symptoms are mitigated or improved, the test substance can be determined as being effective for human fatty liver and can be used for treatment or improvement of the human fatty liver. Since the substance effective for treatment or improvement of a disease is generally effective for the disease, the substance effective for treatment or improvement of human fatty liver is determined as being effective for prevention of human fatty liver. In short, the "substance effective for human fatty liver" means a substance effective for prevention, treatment or improvement of human fatty liver. Examples of the test substance include, but are not limited to, a small molecule compound, an amino acid, a nucleic acid, lipid, sugar and an extract of a natural product.

The human fatty-liver model cells of the present invention can be used in a method for evaluating the toxicity of a test substance to human fatty liver. The toxicity can be evaluated by administering a test substance to a culture of the human fatty-liver model cells of the present invention, comparing the survival rate of cells and severity of fatty-liver symptoms between cells to which the test substance is administered and cells to which the test substance is not administered, to evaluate the effect of the test substance on human fatty liver. In the cells to which the test substance is administered, if the survival rate of cells decreases or the fatty-liver symptoms become severer, the test substance can be determined to have toxicity to human fatty liver. The "decreasing the survival rate of cells" may be determined by counting the number of cultured cells before and after administration of a test substance or based on the content of human albumin secreted in the culture supernatant as an index. If the content of human albumin in the culture supernatant decreases after administration of a test substance compared to that before administration, it is suggested that the survival rate of cells has decreased. In this case, the test substance (or the amount of the test substance) can be determined to have toxicity to human fatty liver. The "cells in which the test substance is administered and not administered", "fatty-liver symptoms" and "test substance" can be the same as defined in the above.

Now, the present invention will be more specifically described by way of Examples; however, the present invention is not limited to these.

### [Examples]

### I. Test method

### 1. Preparation of human hepatocytes derived from fatty liver

### (Preparation of chimeric mice (PXB mice) having human hepatocytes)

PXB mice were prepared in accordance with a method known in the art (Japanese Patent Laid-Open No. 2002-45087). More specifically, a mouse genetically having a liver disorder in which all cells had an introduced urokinase plasminogen activator (uPA) gene (cDNA-uPA) ligated to an enhancer and a promoter of albumin to be synthesized in the liver, was crossed with an immunodeficient mouse (SCID mouse) to prepare immunodeficient mice with liver disorder (cDNA-uPA (+/-)/SCID mice).

The mice (cDNA-uPA (+/-)/SCID) of 3 weeks old were anesthetized. Skin around the spleen and rectus abdominis were cut by scissors. The tip of the spleen was picked up and fixed at the position to facilitate introduction of cells. Subsequently, using a glass syringe filled with a human hepatocyte suspension, human hepatocytes were injected from the tip of the spleen by inserting the needle. Thereafter, the spleen was returned to the mice and the skin and peritoneum were sawed by use of a plastic surgery needle to close the incision site. After confirming no abnormality of breathing of the mice transplanted, the mice were raised in a rearing cage.

PXB mice of 17 to 22 weeks old, which had a body weight of 15 to 20 g and a serum human albumin content of 10 mg/mL or more (replacement rate of human hepatocytes calculated based on the amount of human albumin was 95% or more), were selected and used in the following experiments.

### (Separation of cells)

The PXB mouse under anesthesia was placed on a dissection table, fixed with medical tape, and then, subjected to laparotomy. An intravenous cannula was inserted in the portal vein, perfusate A was fed to remove the blood. Perfusate B was fed to dissolve collagen in the liver tissue and the liver is excised out so as not to damage the intestinal tract and stomach. The liver was shaken in perfusate C to release/separate hepatocytes. Undigested tissue pieces were removed by passing the resultant solution through a cell strainer and the hepatocytes were recovered in a tube.

### (Preparation of cells)

The hepatocytes (PXB-cells) recovered were centrifuged. After the supernatant was removed, 40 mL of medium A was added to the resultant sediment. The mixture was gently stirred. This operation was repeated twice to remove, e.g., impurities and lipid suspended in the supernatant. The resultant solution was passed through a cell strainer to isolate and collect cell mass in a tube. The number of cells was counted in accordance with a trypan blue dye exclusion method by a hemocytometer. Based on the count value, the density, total number and survival rate of cells were obtained.

### (Seeding)

Based on the cell density of the cell suspension, the dilution rate for obtaining a desired seeding density was calculated and the cell suspension was diluted with medium A. To each of the wells of a culture plate, the diluted cell suspension (500 µL) was gently poured. The plate was allowed to stand still for about 20 minutes until the cells were slightly in contact with the bottom surface of the wells and gently placed in an incubator (37°C, 5% CO₂) to culture the cells.

### 2. Analysis of lipoprotein in the culture supernatant of cells cultured in DMSO-containing medium

The following day of seeding, medium A was removed. Instead, 500 µL of medium B (DMSO (+)) or medium C (DMSO (-)) was added. The plate was gently placed in an incubator (37°C, 5% CO₂) and the cells were cultured for 5 days. After completion of culture, an image of the cells was taken by a photomicrographic camera. Then, the culture supernatant was recovered and subjected to analysis for lipoprotein in the culture supernatant described below.

### 3. Analysis of lipoprotein in the culture supernatant of cells cultured in DMSO-containing medium for 5 to 14 days

### • Culture in DMSO-containing medium for 5 days

The following day of seeding, medium A was removed. Instead, 500 µL of medium B (DMSO (+)) was added. The plate was gently placed in an incubator (37°C, 5% CO₂) and the cells were cultured. Day 1 and Day 2 after initiation of culture with medium B (DMSO (+)), the medium was exchanged with fresh medium B (DMSO (+)).

After completion of culture with medium B (DMSO (+)) for 5 days, the following analysis for lipoprotein in the culture supernatant was carried out.

### • Culture in DMSO-containing medium for 9 days

The following day of seeding, medium A was removed. Instead, 500 µL of medium B (DMSO (+)) was added. The plate was gently placed in an incubator (37°C, 5% CO₂) and the cells were cultured. Day 5, Day 7 and Day 8 after initiation of culture with medium B (DMSO (+)), the medium was exchanged with fresh medium B (DMSO (+)).

After completion of culture with medium B (DMSO (+)) for 9 days, the following analysis for lipoprotein in the culture supernatant was carried out.

### • Culture in DMSO-containing medium for 12 days

The following day of seeding, medium A was removed. Instead, 500 µL of medium B (DMSO (+)) was added. The plate was gently placed in an incubator (37°C, 5% CO₂) and the cells were cultured. Day 5, Day 7 and Day 8 after initiation of culture with medium B (DMSO (+)), the medium was exchanged with fresh medium B (DMSO (+)).

After completion of culture with medium B (DMSO (+)) for 12 days, the following analysis for lipoprotein in the culture supernatant was carried out.

### • Culture in DMSO-containing medium for 14 days

The following day of seeding, medium A was removed. Instead, 500 µL of medium B (DMSO (+)) was added. The plate was gently placed in an incubator (37°C, 5% CO₂) and the cells were cultured. Day 5, Day 7, Day 8 and Day 12 after initiation of culture with medium B (DMSO (+)), the medium was exchanged with fresh medium B (DMSO (+)).

After completion of culture with medium B (DMSO (+)) for 14 days, the following analysis for lipoprotein in the culture supernatant was carried out.

### 4. Analysis of lipoprotein in the cells cultured in DMSO-containing medium for 5 to 14 days

### • Culture in DMSO-containing medium for 5 days

The following day of seeding, medium A was removed. Instead, 500 µL of medium B (DMSO (+)) was added. The plate was gently placed in an incubator (37°C, 5% CO₂) and the cells were cultured. Day 1 and Day 2 after initiation of culture with medium B (DMSO (+)), the medium was exchanged with fresh medium B (DMSO (+)).

After completion of culture with medium B (DMSO (+)) for 5 days, the following analysis for intracellular lipoproteins was carried out for the contents of cholesterol and triglyceride in the cells.

### • Culture in DMSO-containing medium for 9 days

The following day of seeding, medium A was removed. Instead, 500 µL of medium B (DMSO (+)) was added. The plate was gently placed in an incubator (37°C, 5% CO₂) and the cells were cultured. Day 5, Day 7 and Day 8 after initiation of culture with medium B (DMSO (+)), the medium was exchanged with fresh medium B (DMSO (+)).

After completion of culture with medium B (DMSO (+)) for 9 days, the following analysis for intracellular lipoproteins was carried out for the contents of cholesterol and triglyceride in the cells.

### • Culture in DMSO-containing medium for 12 days

The following day of seeding, medium A was removed. Instead, 500 µL of medium B (DMSO (+)) was added. The plate was gently placed in an incubator (37°C, 5% CO₂) and the cells were cultured. Day 5, Day 7 and Day 8 after initiation of culture with medium B (DMSO (+)), the medium was exchanged with fresh medium B (DMSO (+)).

After completion of culture with medium B (DMSO (+)) for 12 days, the following analysis for intracellular lipoproteins was carried out for the contents of cholesterol and triglyceride.

### • Culture in DMSO-containing medium for 14 days

The following day of seeding, medium A was removed. Instead, 500 µL of medium B (DMSO (+)) was added. The plate was gently placed in an incubator (37°C, 5% CO₂) and the cells were cultured. Day 5, Day 7, Day 8 and Day 12 after initiation of culture with medium B (DMSO (+)), the medium was exchanged with fresh medium B (DMSO (+)).

After completion of culture with medium B (DMSO (+)) for 14 days, the following analysis for intracellular lipoproteins was carried out for the contents of cholesterol and triglyceride.

### • Culture of hepatocytes known in the art

Hepatocytes (HepG2 cells, HuH7 cells) known in the art were seeded in 500 µL of medium A, gently placed in an incubator (37°C, 5% CO₂) and cultured for 7 days. After completion of culture, the following analyses for lipoproteins in the culture supernatant and within the cells, were carried out.

### 5. Analysis method

### (Analysis of lipoprotein in culture supernatant)

Lipoproteins contained in the culture supernatants of PXB-cells, HepG2 cells and HuH7 cells were analyzed by using LipoSEARCH (registered trademark) method (Skylight Biotech, Inc.).

After completion of the culture mentioned above, the medium used in the culture was removed and 500 µL of medium D for analysis was added. The culture plate was gently placed in an incubator (37°C, 5% CO₂) and the cells were cultured for 2 days. Thereafter, the culture supernatant was recovered, lipoproteins contained in the culture supernatant (80 µL) were fractionated into 4 types of subgroups, CM, VLDL, LDL and HDL fractions, by gel filtration HPLC. Cholesterol and neutral fat (triglyceride) contained in individual fractions were quantified by online enzyme reactions. Concentration analysis was carried out in accordance with the computer program specifically developed by Skylight Biotech, Inc. Note that, in the enzymatic reaction, Diacolor Liquid TG-S (manufactured by Toyobo Co., Ltd.) was used. As standard sera for cholesterol and neutral fat (triglyceride) concentration in CM, VLDL, LDL and HDL, the sera manufactured by Kyowa Hakko Kirin Co., Ltd. were used.

### (Analysis for lipoprotein in cells)

### • Measurement of triglyceride in cells

Triglyceride in cells was measured by use of Cholestest (registered trademark) TG (Sekisui Medical Co., Ltd.) in accordance with the instruction by the manufacturer. More specifically, the cells were washed with PBS, and then, completely dewatered (stored at -80 °C until measurement). To each well containing the cells, 200 µL of TG enzyme solution (1) was added. A reaction was allowed to proceed while keeping the cells warm at 37°C for 10 minutes (free glycerol was removed). Then, the cells were torn away by pipetting, transferred to a centrifuge tube and centrifuged at 10,000 rpm × 10 minutes. Then, the supernatant (7.5 µL) was transferred to a 96-well microplate. To this, 68 µL of TG enzyme solution (1) was added and the microplate was kept warm at 37°C for 10 minutes to remove completely free glycerol. Then, 25 µL of TG enzyme solution (2) was added and kept the plate warm at 37°C for 10 minutes. The resultant reaction product was subjected to measurement of absorbance at 550 nm. The content of triglyceride was calculated based on HDL-C180A as the reference (triglyceride concentration of HDL-C180A was 52.26 mg/dL).

### • Measurement of intracellular cholesterol

Intracellular cholesterol was measured by use of Cholestest (registered trademark) CHO (Sekisui Medical Co., Ltd.) in accordance with the instruction by the manufacturer. More specifically, the cells were washed with PBS, and then, completely dewatered (stored at -80 °C until measurement). To each well containing the cells, 200 µL of CHO enzyme solution (1) was added and kept the cells warm at 37°C for 10 minutes. Then, the cells were torn away by pipetting, transferred to a centrifuge tube and centrifuged at 10,000 rpm × 10 minutes. Then, the supernatant (15 µL) was transferred to a 96-well microplate. To this, 68 µL of CHO enzyme solution (1) was added and kept warm at 37°C for 10 minutes. Subsequently, 25 µL of CHO enzyme solution (2) was added to the wells and kept the plate warm at 37°C for 10 minutes. The resultant reaction product was subjected to measurement of absorbance at 550 nm. The content of cholesterol was calculated based on HDL-C180A as the standard (triglyceride concentration of HDL-C180A was 152.67 mg/dL).

### 6. Analysis for gene expression level

The total RNA of PXB-cells cultured in medium B for 3 days or 6 days was extracted by use of TRIzol (registered trademark) + Direct zol (Thermo Fisher Scientific k.k.) in accordance with the instruction by the manufacturer.

After the quality of the total extracted RNA sample was evaluated by a bioanalyzer (Agilent Technologies, Inc.), microarray analysis (Agilent Technologies, Inc.) was carried out in accordance with the instruction by the manufacturer to analyze the expression levels of fatty liver related genes, FASN, SREBF1, G6PC, CYP7A1, CETP, GCK and PCK1.

### 7. Perfusate, medium

The compositions of perfusate A, perfusate B, perfusate C, medium A, medium B, medium C and medium D used herein are as follows.

**[Table 2]**

| Perfusate A | |
|---|---|
| Name of reagent | Final concentration |
| HBSS(-) | --- |
| D-glucose | 1 mg/mL |
| EGTA | 200 µg/mL |
| 50 mg/mL Gentamycin | 10 µg/mL |
| 1M HEPES buffer | 10 mM |

| Perfusate B | |
|---|---|
| Name of reagent | Final concentration |
| HBSS(-) | --- |
| Type IV collagenase | 0.05% |
| CaCl₂ | 600 µg/mL |
| 50 mg/mL Gentamycin | 10 µg/mL |
| 1M HEPES buffer | 10 mM |
| Trypsin inhibitor | 100 µg/mL |

| Perfusate C | |
|---|---|
| Name of reagent | Final concentration |
| HBSS(-) | --- |
| 10% Albumin solution | 10 mg/mL |
| 50 mg/mL Gentamycin | 10 µg/mL |
| 1M HEPES buffer | 10 mM |

**[Table 3]**

| Medium A | |
|---|---|
| Reagent | Final concentration |
| Dulbecco's modified eagle medium | - |
| NaHCO₃ | 44 mM |
| Penicillin G | 100 IU mL |
| Streptomycin | 100 µg/mL |
| N-2-Hydroxyethylpiperazine-N-2-ethanesulfonic acid (HEPES) | 20 mM |
| Fetal bovine serum (FBS) | 10% |

**[Table 4]**

| Medium B | |
|---|---|
| Reagent | Final concentration |
| Dulbecco's modified eagle medium | - |
| NaHCO₃ | 44 mM |
| Penicillin G | 100 IU mL |
| Streptomycin | 100 µg/mL |
| HEPES | 20 mM |
| FBS | 10% |
| L-Proline | 15 µg/mL |
| Insulin | 0.25 µg/mL |
| Dexamethasone | 50 nM |
| Epidermal growth factor (EGF) | 5 ng/mL |
| L-Ascorbic acid 2-phosphate (Asc-2P) | 0.1 mM |
| Dimethyl sulfoxide (DMSO) | 2% |

| Medium C (Medium B minus DMSO) | |
|---|---|
| Reagent | Final concentration |
| Dulbecco's modified eagle medium | - |
| NaHCO₃ | 44 mM |
| Penicillin G | 100 IU mL |
| Streptomycin | 100 µg/mL |
| HEPES | 20 mM |
| FBS | 10% |
| L-Proline | 15 µg/mL |
| Insulin | 0.25 µg/mL |
| Dexamethasone | 50 nM |
| EGF | 5 ng/mL |
| Asc-2P | 0.1 mM |

| Medium D | |
|---|---|
| Reagent | Final concentration |
| Williams medium E | - |
| CM4000 | Concentration recommended by manufacturer |

### II. Results

### 1. Analysis results of lipoprotein contained in the culture supernatant of cells cultured in DMSO-containing medium

Figure 1 shows PXB-cells cultured separately in medium B (DMSO (+)) and medium C (DMSO (-)) for 5 days. In the PXB-cells cultured in medium B (DMSO (+))(Figure 1, left) compared to PXB cells cultured in medium C (DMSO (-))(Figure 1, right), a large number (about double) of fat droplets (observed as white) were observed.

Figure 2 shows the measurement results of the content of total neutral fat (triglyceride) of lipoproteins (including CM, VLDL, LDL and HDL) contained in the culture supernatants recovered after culture in each of medium B (DMSO (+)) and medium C (DMSO (-)) for 5 days. The results are shown by relative values based on the content (regarded as "100") of the total neutral fat in the culture supernatant of PXB-cells cultured in medium B (DMSO (+)). It was confirmed that secretion of the total neutral fat (triglyceride) in lipoproteins contained in PXB-cells cultured in medium B (DMSO (+)) is about 9 times as high as that cultured in medium C (DMSO (-)).

From the results, it was confirmed that if PXB-cells are cultured in a DMSO-containing medium, accumulation and secretion of lipid can be kept at a high level.

### 2. Analysis results of lipoprotein contained in the culture supernatant of cells cultured in DMSO-containing medium for 5 to 14 days

Figure 3 shows analysis results of lipoproteins in the culture supernatants of PXB-cells in medium B (DMSO (+)) for 5, 9, 12 and 14 days, and hepatocytes known in the art (HepG2 cells, HuH7 cells).

In PXB-cells, the total cholesterol and total neutral fat (triglyceride) contents in the culture supernatant were both the highest in the case of culturing the cells for 5 days. In the cases of culturing the cells for 9 days, 12 days and 14 days, the contents thereof were maintained at slightly lower levels than this.

In contrast, it was confirmed that the total cholesterol and total neutral fat (triglyceride) contents in the culture supernatant of HepG2 cells are both significantly low compared to those in the culture supernatant of PXB-cells. It was also confirmed that the total cholesterol content in the culture supernatant of HuH7 cells is equivalent to those of PXB-cells cultured for 12 days and 14 days; however, the content of the total neutral fat (triglyceride) is remarkably low compared to that of PXB-cells.

Figure 4 shows the analysis results of lipoproteins (4 types of subgroups) contained in the culture supernatant. In the PXB-cells cultured in medium B (DMSO (+)), it was confirmed that the content of VLDL is the largest in each of the cholesterol and neutral fat (triglyceride), regardless of the culture period. The analysis results of lipoproteins (content ratio (weight ratio) of CM, VLDL, LDL, HDL) in the culture supernatant of PXB-cells cultured in medium B (DMSO (+)) for 5 days, 9 days, 12 days and 14 days will be described below.

### • Culture for 5 days

(Cholesterol)
   CM: VLDL: LDL: HDL = 3: 86: 8: 3
(Neutral fat (triglyceride))
   CM: VLDL: LDL: HDL = 3: 91: 5: 1

### • Culture for 9 days

(Cholesterol)
   CM: VLDL: LDL: HDL = 4: 82: 9: 2
(Neutral fat (triglyceride))
   CM: VLDL: LDL: HDL = 3: 90: 6: 1

### • Culture for 12 days

(Cholesterol)
   CM: VLDL: LDL: HDL = 2: 80: 9: 3
(Neutral fat (triglyceride))
   CM: VLDL: LDL: HDL = 3: 90: 6: 1

### • Culture for 14 days

(Cholesterol)
   CM: VLDL: LDL: HDL = 1: 79: 14: 6
(Neutral fat (triglyceride))
   CM: VLDL: LDL: HDL = 2: 91: 6: 1

In contrast, as to HuH7 cells, the content of LDL was the highest in each of cholesterol and neutral fat (triglyceride) contained in the culture supernatant thereof. As to HepG2 cells cultured in the same condition, the content of HDL was the highest in each of cholesterol and neutral fat (triglyceride) contained in the culture supernatant thereof.

The peak of VLDL was observed in PXB-cells and not observed in culture supernatants of HepG2 cells and HuH7 cells. It was confirmed that the VLDL is secreted specifically in PXB-cells cultured in medium B (DMSO (+)).

### 3. Analysis results of lipoprotein in the cells cultured in DMSO-containing medium for 5 to 14 days

Figure 5 shows the analysis results of lipoproteins in each of PXB-cells cultured in medium B (DMSO (+)) for 5, 9, 12 and 14 days and hepatocytes (HepG2 cells, HuH7 cells) known in the art.

In PXB-cells, the content of the total cholesterol was the lowest after culture for 5 days and slightly higher after culture for 9 days, 12 days and 14 days. The content of the total neutral fat (triglyceride) was the highest after culture for 5 days and maintained at a slightly lower level after culture for 9 days, 12 days and 14 days.

In contrast, it was confirmed that the total cholesterol and total neutral fat (triglyceride) contents in HepG2 cells are both remarkably low compared to those in PXB-cells. It was also confirmed that the total neutral fat (triglyceride) content in HuH7 cells is equivalent to those of PXB-cells after culture for 9 days, 12 days and 14 days but is remarkably lower than that of PXB-cells after culture for 5 days. It was confirmed that the total cholesterol is equivalent to those of the all periods of PXB-cells.

From these results, it was confirmed that PXB-cells, which were cultured in medium B (DMSO (+)), can maintain accumulation and secretion of lipid (cholesterol and neutral fat (triglyceride)) at least about two weeks. Particularly, in the culture for 6 days, the highest accumulation of neutral fat (triglyceride)) and secretion of cholesterol and neutral fat (triglyceride) were confirmed. It was further confirmed that the secreted lipoproteins contain VLDL in the largest amount. Such feature is not observed in hepatocytes (HuH7 cells, HepG2 cells) previously known in the art. It was demonstrated that PXB-cells cultured in the medium B (DMSO (+)) have different properties from those of hepatocytes previously known in the art.

### 4. Expression level of the fatty liver related gene

Figure 6 shows the analysis results of expression levels of fatty liver related genes (FASN, SREBF1, G6PC) in PXB-cells cultured in medium B (DMSO (+)) for 3 days and 6 days. The results are shown by relative values based on the expression level (regarded as "1") of each gene on Day 3 after initiation of culture. In any one of the genes, the expression level increased on Day 6 from Day 3 after initiation of culture. Although not shown in Figure 6, the expression levels of CYP7A1, CETP, GCK and PCK1 similarly increased on Day 6 from Day 3 after initiation of culture.

From the above results, it was confirmed that accumulation and secretion of lipid can be maintained in PXB-cells cultured in medium B (DMSO (+)) and also fatty liver related genes are expressed. From this, it was demonstrated that PXB-cells can be used as human fatty-liver model cells.

### III. Screening for substance effective for prevention, treatment or improvement of human fatty liver

### 1. Test method

### • Preparation of cells

PXB-cells were diluted with medium A. The diluted cell suspension (500 µL) was gently poured to individual wells of a culture plate. The plate was allowed to stand still for about 20 minutes until the cells were slightly in contact with the bottom surface of the wells and gently placed to an incubator (37°C, 5% CO₂) to culture the cells. The following day of seeding, medium A was removed and 500 µL of medium B (DMSO (+)) was added. The plate was gently placed in an incubator (37°C, 5% CO₂) and the cells were cultured for 5 days. Also, cells to which an antihyperlipidemic drug was administered were cultured for 12 days. After completion of culture, the cells were used in the following screening test.

### • Screening test

As a test substance, a junsai extract (Oryza Oil&Fat Chemical Co., Ltd.) known as a lipid metabolism improver (Patent No. 5344494) was used. Also simvastatin (FUJIFILM Wako Pure Chemical Corporation), fenofibrate (Sigma-Aldrich Co. LLC.) and lomitapide (Tokyo Chemical Industry Co., Ltd.) serving as an antihyperlipidemic drug were used as test substances.

Each test substance was suspended with ethanol and added in a predetermined amount to a PXB-cell culture, gently placed in an incubator (37°C, 5% CO₂) and cultured for 2 days. After completion of culture, the total cholesterol and total neutral fat (triglyceride) in culture supernatant and in cells were measured in accordance with the methods described in the sections "Analysis of lipoprotein in culture supernatant" and "Analysis of lipoprotein in cells".

To a control sample, only the same amount of ethanol as used for suspending a test substance, was added.

### • Measurement of albumin

After completion of culture, the culture supernatant (200 µL) was taken and subjected to measurement of the human albumin content in the culture supernatant performed by an automatic analyzer JCA-BM6050 (JEOL Ltd.) in accordance with an immunoturbidimetric method.

### 2. Results

Figure 7 shows the analysis results of the contents of the lipoproteins in cells and in the culture supernatants of PXB-cells cultured in mediums respectively supplemented with a junsai extract (5 µg/mL, 50 µg/mL and 500 µg/mL) for 2 days, and the contents of human albumin in individual culture supernatants.

When a junsai extract was added, no significant decrease in total cholesterol content was observed in the culture supernatants and in cells in any addition amount. No significant decrease was observed in the content of the total neutral fat (triglyceride) in the culture supernatants; however, the content of the total neutral fat (triglyceride) in the cells decreased depending on the addition amount of the junsai extract.

No significant decrease in the content of human albumin in the culture supernatant was observed in any addition amount. Toxicity was not confirmed.

Figure 8 shows the analysis results of the contents of lipoproteins in cells and in the culture supernatants of PXB-cells cultured in mediums respectively supplemented with simvastatin (0.1 µM, 1 µM and 10 µM) for 2 days and the content of human albumin in individual culture supernatants.

When simvastatin was added, no significant decrease of the total neutral fat (triglyceride) content was observed in the culture supernatants and in cells in any addition amount. In contrast, although no significant decrease of the total cholesterol content was observed in cells; the total cholesterol content in the culture supernatants decreased depending on the addition amount of simvastatin.

Also no significant decrease of human albumin content in the culture supernatant was observed in any addition amount. Toxicity was not confirmed.

Figure 9 shows the analysis results of the contents of lipoproteins in cells and in the culture supernatants of PXB-cells cultured in mediums respectively supplemented with fenofibrate (5 µM, 50 µM and 500 µM) for 2 days and the content of human albumin in individual culture supernatants.

When fenofibrate was added, no significant decrease of the total cholesterol content and total neutral fat (triglyceride) content in the cells was observed in any addition amount. In contrast,
the total cholesterol content and total neutral fat (triglyceride) content in the culture supernatant, decreased depending on the addition amount of fenofibrate.

Also the content of human albumin in the culture supernatant decreased depending on the addition amount of fenofibrate. It was suggested that a high amount of fenofibrate is toxic.

Figure 10 shows the analysis results of lipoprotein contents in cells and in individual culture supernatants of PXB-cells cultured in mediums respectively supplemented with lomitapide (1 µM, 10 µM and 100 µM) for 2 days and the contents of human albumin in individual culture supernatants.

When lomitapide was added, no significant decrease of the total cholesterol content and total neutral fat (triglyceride) content in the cells was observed in any addition amount. In contrast,
the total cholesterol content and total neutral fat (triglyceride) content in the culture supernatants were significantly decreased by addition of lomitapide.

Also the content of human albumin in the culture supernatant decreased depending on the addition amount of lomitapide. It was suggested that a high amount of lomitapide is toxic.

From the above results, it was demonstrated that PXB-cells can be used for screening for a substance effective for prevention, treatment or improvement of human fatty liver, such as a lipid metabolism improver and an antihyperlipidemic drug, based on a decrease of the total cholesterol content and total neutral fat content in the culture supernatant and/or in cells. It was also demonstrated that PXB-cells can be used for evaluating and determining a substance that may be toxic to human fatty liver, based on a decrease of the human albumin content in the culture supernatant thereof.

## Claims

1. A method for producing human fatty-liver model cells, comprising a step of culturing human hepatocytes derived from fatty liver in a medium containing dimethyl sulfoxide.

2. The method according to claim 1, wherein the human hepatocytes derived from fatty liver are collected from a chimeric non-human animal having human hepatocytes.

3. The method according to claim 1 or 2, wherein the culturing is carried out for more than 3 days.

4. Human fatty-liver model cells that secrete and/or accumulate lipid.

5. The cells according to claim 4, comprising a lipoprotein including a very low density lipoprotein (VLDL) and a low density lipoprotein (LDL), wherein VLDL is comprised more than LDL.

6. The cells according to claim 4, having increased expression of a fatty liver related gene.

7. The cells according to claim 6, wherein the fatty liver related gene is at least one gene selected from the group consisting of FASN, SREBF1 and G6PC.

8. A method for screening for a substance effective for human fatty liver, comprising the steps of:
administering a test substance to the cells according to any one of claims 4 to 7; and
comparing severity of fatty-liver symptoms between cells to which the test substance is administered and cells to which the test substance is not administered.

9. A method for evaluating toxicity of a test substance to human fatty liver, comprising the steps of:
administering a test substance to the cells according to any one of claims 4 to 7; and
comparing survival rate and severity of fatty-liver symptoms between cells to which the test substance is administered and cells to which the test substance is not administered, to evaluate effect of the test substance on human fatty liver.
